# EUROPEAN PATENT SPECIFICATION

(11) **EP 3 622 407 B1**
(45) Date of publication and mention of the grant of the patent: **05.07.2023**
(21) Application number: 18799372.0
(22) Date of filing: 03.05.2018
(51) Int. Cl.: G16H 50/20

(54) **PATIENT TREATMENT SYSTEMS AND METHODS**
SYSTEME UND VERFAHREN ZUR PATIENTENBEHANDLUNG
SYSTÈMES ET MÉTHODES DE TRAITEMENT DE PATIENTS

(30) Priority: 09.05.2017 US 201715590997
(43) Date of publication of application: 18.03.2020
(73) Proprietor: AdviNow, Inc., Paradise Valley, AZ 85253 (US)
(72) Inventor: BATES, James Stewart, Paradise Valley, Arizona 85253 (US); PHILLIPS, Julian, Phoenix, Arizona 85006 (US)
(74) Representative: VKK Patentanwälte PartG mbB
(86) International application number: PCT/US2018/030991
(87) International publication number: WO 2018/208581

(56) References cited:
- EP-A2- 1 352 356
- US-A1- 2009 299 766
- US-A1- 2012 084 092
- US-A1- 2012 232 930
- US-A1- 2014 095 201
- US-A1- 2014 279 746
- US-A1- 2015 111 220
- US-A1- 2016 038 092
- HIDALGO SEBASTIAN J ET AL: "A Master Pipeline for Discovery and Validation of Biomarkers", 10 December 2016 (2016-12-10), BIG DATA ANALYTICS IN THE SOCIAL AND UBIQUITOUS CONTEXT : 5TH INTERNATIONAL WORKSHOP ON MODELING SOCIAL MEDIA, MSM 2014, 5TH INTERNATIONAL WORKSHOP ON MINING UBIQUITOUS AND SOCIAL ENVIRONMENTS, MUSE 2014 AND FIRST INTERNATIONAL WORKSHOP ON MACHINE LE, XP047365025, ISBN: 978-3-642-17318-9 [retrieved on 2016-12-10] * the whole document *

## Description

### Technical Field

The present disclosure relates to health care, and more particularly, to systems and methods for providing doctors and patients with treatment suggestions based on computer-aided diagnoses.

### Background of the Invention

Patients' common problems with scheduling an appointment with a primary doctor when needed or in a time-efficient manner is causing a gradual shift away from patients establishing and relying on a life-long relationship with a single general practitioner, who diagnoses and treats a patient in health-related matters, towards patients opting to receive readily available treatment in urgent care facilities that are located near home, work, or school and provide relatively easy access to health care without the inconvenience of appointments that oftentimes must be scheduled weeks or months ahead of time. Yet, the decreasing importance of primary doctors makes it difficult for different treating physicians to maintain a reasonably complete medical record for each patient, which results in a patient having to repeat a great amount of information personal and medical each time when visiting a different facility or different doctor. In some cases, patients confronted with lengthy and time-consuming patient questionnaires fail to provide accurate information that may be important for a proper medical treatment, whether for the sake of expediting their visit or other reasons. In addition, studies have shown that patients attending urgent care or emergency facilities may in fact worsen their health conditions due to the risk of exposure to bacteria or viruses in medical facilities despite the medical profession's efforts to minimize the number of such instances.

Through consistent regulation changes, electronic health record changes and pressure from payers, both health care facilities and providers are looking for ways to make patient intake, triage, diagnosis, treatment, electronic health record data entry, treatment, billing, and patient follow-up activity more efficient, provide better patient experience, and increase the doctor to patient throughput per hour, while simultaneously reducing cost.

The desire to increase access to health care providers, a pressing need to reduce health care costs in developed countries and the goal of making health care available to a larger population in less developed countries have fueled the idea of telemedicine. In most cases, however, video or audio conferencing with a doctor does not provide sufficient patient-physician interaction that is necessary to allow for a proper medical diagnosis to efficiently serve patients.

What is needed are systems and methods that ensure reliable remote or local medical patient intake, triage, diagnosis, treatment, electronic health record data entry/management, treatment, billing and patient follow-up activity so that physicians can allocate patient time more efficiently and, in some instances, allow individuals to manage their own health, thereby, reducing health care costs.

Attempts have been made to satisfy the needs, wherein details can be found in documents US 2012 084 092 A1, US 2015 111 220 A1, US 2016 038 092 A1, US 2012 232 930 A1, Hidalgo et. Al., "A Master Pipeline for Discovery and Validation of Biomarkers", and EP 1 352 356 A0.

An object of the invention is to provide a medical data system and method for automatically generating treatment instructions with increased reliability and minimum costs.

The object is satisfied by the subject-matters of the independent claims.

### BRIEF DESCRIPTION OF THE DRAWINGS

References will be made to embodiments of the invention, examples of which may be illustrated in the accompanying figures. These figures are intended to be illustrative, not limiting. Although the invention is generally described in the context of these embodiments, it should be understood that it is not intended to limit the scope of the invention to these particular embodiments.
**FIG. 1** illustrates an exemplary diagnostic system according to embodiments of the present disclosure.
**FIG. 2** illustrates an exemplary medical instrument equipment system according to embodiments of the present disclosure.
**FIG. 3** illustrates an exemplary medical data system according to embodiments of the present disclosure.
**FIG. 4** is a flowchart of an illustrative process for generating patient treatment information according to embodiments of the present disclosure.
**FIG. 5** depicts a simplified block diagram of a computing device /information handling system according to embodiments of the present disclosure.

In the following description, for purposes of explanation, specific details are set forth in order to provide an understanding of the disclosure. It will be apparent, however, to one skilled in the art that the disclosure can be practiced without these details. Furthermore, one skilled in the art will recognize that embodiments of the present disclosure, described below, may be implemented in a variety of ways, such as a process, an apparatus, a system, a device, or a method on a tangible computer-readable medium.

Elements/components shown in diagrams are illustrative of exemplary embodiments of the disclosure and are meant to avoid obscuring the disclosure. It shall also be understood that throughout this discussion that components may be described as separate functional units, which may comprise sub-units, but those skilled in the art will recognize that various components, or portions thereof, may be divided into separate components or may be integrated together, including integrated within a single system or component. It should be noted that functions or operations discussed herein may be implemented as components/elements. Components/elements may be implemented in software, hardware, or a combination thereof.

Furthermore, connections between components or systems within the figures are not intended to be limited to direct connections. Rather, data between these components may be modified, re-formatted, or otherwise changed by intermediary components. Also, additional or fewer connections may be used. Also, additional or fewer connections may be used. It shall also be noted that the terms "coupled" "connected" or "communicatively coupled" shall be understood to include direct connections, indirect connections through one or more intermediary devices, and wireless connections.

Reference in the specification to "one embodiment," "preferred embodiment," "an embodiment," or "embodiments" means that a particular feature, structure, characteristic, or function described in connection with the embodiment is included in at least one embodiment of the disclosure and may be in more than one embodiment. The appearances of the phrases "in one embodiment," "in an embodiment," or "in embodiments" in various places in the specification are not necessarily all referring to the same embodiment or embodiments. The terms "include," "including," "comprise," and "comprising" shall be understood to be open terms and any lists that follow are examples and not meant to be limited to the listed items. Any headings used herein are for organizational purposes only and shall not be used to limit the scope of the description or the claims. The embodiments of the invention for which protection is sought are defined by the claims.

Furthermore, the use of certain terms in various places in the specification is for illustration and should not be construed as limiting. A service, function, or resource is not limited to a single service, function, or resource; usage of these terms may refer to a grouping of related services, functions, or resources, which may be distributed or aggregated.

In this document, the term "sensor" refers to a device capable of acquiring information related to any type of physiological condition or activity (e.g., a biometric diagnostic sensor); physical data (e.g., a weight); and environmental information (e.g., ambient temperature sensor), including hardware-specific information. The term "position" refers to spatial and temporal data (e.g., orientation and motion information). "Doctor" refers to any health care professional, health care provider, physician, or person directed by a physician. "Patient" is any user who uses the systems and methods of the present invention, e.g., a person being examined or anyone assisting such person. The term illness may be used interchangeably with the term diagnosis. As used herein, "answer" or "question" refers to one or more of 1) an answer to a question, 2) a measurement or measurement request (e.g., a measurement performed by a "patient"), and 3) a symptom (e.g., a symptom selected by a "patient").

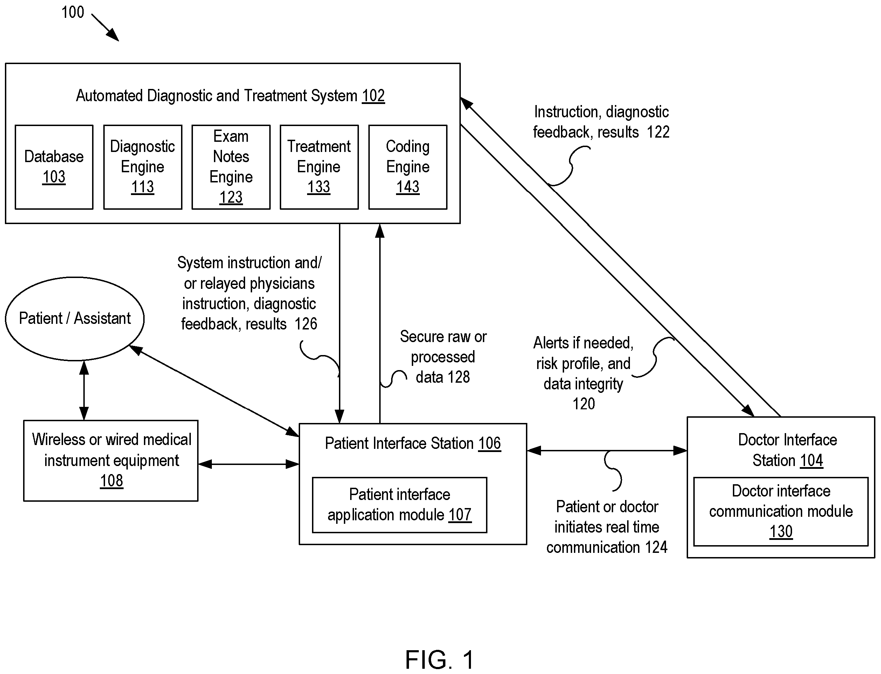

**FIG. 1** illustrates an exemplary diagnostic system according to embodiments of the present disclosure. Diagnostic system **100** comprises automated diagnostic and treatment system **102,** patient interface station **106,** doctor interface station **104,** and medical instrument equipment **108.** Automated diagnostic and treatment system **102** may further comprise database **103,** diagnostic engine **113,** exam notes engine **123,** treatment engine **133,** and coding engine **143.** Both patient interface station **106** and doctor interface station **104** may be implemented into any tablet, computer, mobile device, or other electronic device. Medical instrument equipment **108** is designed to collect mainly diagnostic patient data, and may comprise one or more diagnostic devices, for example, in a home diagnostic medical kit that generates diagnostic data based on physical and non-physical characteristics of a patient. It is noted that diagnostic system **100** may comprise additional sensors and devices that, in operation, collect, process, or transmit characteristic information about the patient, medical instrument usage, orientation, environmental parameters such as ambient temperature, humidity, location, and other useful information that may be used to accomplish the objectives of the present invention.

In operation, a patient may enter patient-related data, such as health history, patient characteristics, symptoms, health concerns, medical instrument measured diagnostic data, images, and sound patterns, or other relevant information into patient interface station **106.** The patient may use any means of communication, such as voice control, to enter data, e.g., in the form of a questionnaire. Patient interface station **106** may provide the data raw or in processed form to automated diagnostic and treatment system **102,** e.g., via a secure communication.

In embodiments, the patient may be prompted, e.g., by a software application, to answer questions intended to aid in the diagnosis of one or more medical conditions. The software application may provide guidance by describing how to use medical instrument equipment **108** to administer a diagnostic test or how to make diagnostic measurements for any particular device that may be part of medical instrument equipment **108** so as to facilitate accurate measurements of patient diagnostic data.

In embodiments, the patient may use medical instrument equipment **108** to create a patient health profile that serves as a baseline profile. Gathered patient-related data may be securely stored in database **103** or a secure remote server (not shown) coupled to automated diagnostic and treatment system **102.** In embodiments, automated diagnostic and treatment system **102** enables interaction between a patient and a remotely located health care professional, who may provide instructions to the patient, e.g., by communicating via the software application. A doctor may log into a cloud-based system (not shown) to access patient-related data via doctor interface station **104.** In embodiments, automated diagnostic and treatment system **102** presents automated diagnostic suggestions to a doctor, who may verify or modify the suggested information.

In embodiments, based on one more patient questionnaires, data gathered by medical instrument equipment **108,** patient feedback, and historic diagnostic information, the patient may be provided with instructions, feedback, results **122,** and other information pertinent to the patient's health. In embodiments, the doctor may select an illness based on automated diagnostic system suggestions and/or follow a sequence of instructions, feedback, and/or results **122** may be adjusted based on decision vectors associated with a medical database. In embodiments, medical instrument equipment **108** uses the decision vectors to generate a diagnostic result, e.g., in response to patient answers and/or measurements of the patient's vital signs.

In embodiments, medical instrument equipment **108** comprises a number of sensors, such as accelerometers, gyroscopes, pressure sensors, cameras, bolometers, altimeters, IR LEDs, and proximity sensors that may be coupled to one or more medical devices, e.g., a thermometer, to assist in performing diagnostic measurements and/or monitor a patient's use of medical instrument equipment **108** for accuracy. A camera, bolometer, or other spectrum imaging device (e.g. radar), in addition to taking pictures of the patient, may use image or facial recognition software and machine vision to recognize body parts, items, and actions to aid the patient in locating suitable positions for taking a measurement on the patient's body, e.g., by identifying any part of the patient's body as a reference.

Examples of the types of diagnostic data that medical instrument equipment **108** may generate comprise body temperature, blood pressure, images, sound, heart rate, blood oxygen level, motion, ultrasound, pressure or gas analysis, continuous positive airway pressure, electrocardiogram, electroencephalogram, Electrocardiography, BMI, muscle mass, blood, urine, and any other patient-related data **128.** In embodiments, patient-related data **128** may be derived from a non-surgical wearable or implantable monitoring device that gathers sample data.

In embodiments, an IR LED, proximity beacon, or other identifiable marker (not shown) may be attached to medical instrument equipment **108** to track the position and placement of medical instrument equipment **108.** In embodiments, a camera, bolometer, or other spectrum imaging device uses the identifiable marker as a control tool to aid the camera or the patient in determining the position of medical instrument equipment **108.**

In embodiments, machine vision software may be used to track and overlay or superimpose, e.g., on a screen, the position of the identifiable marker e.g., IR LED, heat source, or reflective material with a desired target location at which the patient should place medical instrument equipment **108,** thereby, aiding the patient to properly place or align a sensor and ensure accurate and reliable readings. Once medical instrument equipment **108,** e.g., a stethoscope is placed at the desired target location on a patient's torso, the patient may be prompted by optical or visual cues to breath according to instructions or perform other actions to facilitate medical measurements and to start a measurement.

In embodiments, one or more sensors that may be attached to medical instrument equipment **108** monitor the placement and usage of medical instrument equipment **108** by periodically or continuously recording data and comparing measured data, such as location, movement, and angles, to an expected data model and/or an error threshold to ensure measurement accuracy. A patient may be instructed to adjust an angle, location, or motion of medical instrument equipment **108,** e.g., to adjust its state and, thus, avoid low-accuracy or faulty measurement readings. In embodiments, sensors attached or tracking medical instrument equipment **108** may generate sensor data and patient interaction activity data that may be compared, for example, against an idealized patient medical instrument equipment usage sensor model data to create an equipment usage accuracy score. The patient medical instrument equipment measured medical data may also be compared with idealized device measurement data expected from medical instrument equipment **108** to create a device accuracy score.

Feedback from medical instrument equipment **108** (e.g., sensors, proximity, camera, etc.) and actual device measurement data may be used to instruct the patient to properly align medical instrument equipment **108** during a measurement. In embodiments, medical instrument equipment type and sensor system monitoring of medical instrument equipment **108** patient interaction may be used to create a device usage accuracy score for use in a medical diagnosis algorithm. Similarly, patient medical instrument equipment measured medical data may be used to create a measurement accuracy score for use by the medical diagnostic algorithm.

In embodiments, machine vision software may be used to show on a monitor an animation that mimics a patient's movements and provides detailed interactive instructions and real-time feedback to the patient. This aids the patient in correctly positioning and operating medical instrument equipment **108** relative to the patient's body so as to ensure a high level of accuracy when using medical instrument equipment **108** is operated.

In embodiments, once automated diagnostic and treatment system **102** detects unexpected data, e.g., data representing an unwanted movement, location, measurement data, etc., a validation process comprising a calculation of a trustworthiness score or reliability factor is initiated in order to gauge the measurement accuracy. Once the accuracy of the measured data falls below a desired level, the patient may be asked to either repeat a measurement or request assistance by an assistant, who may answer questions, e.g., remotely via an application to help with proper equipment usage, or alert a nearby person to assist with using medical instrument equipment **108.** The validation process, may also instruct a patient to answer additional questions, and may comprise calculating the measurement accuracy score based on a measurement or re-measurement.

In embodiments, upon request **124,** automated diagnostic and treatment system **102** may enable a patient-doctor interaction by granting the patient and doctor access to diagnostic system **100.** The patient may enter data, take measurements, and submit images and audio files or any other information to the application or web portal. The doctor may access that information, for example, to review a diagnosis generated by automated diagnostic and treatment system **102,** and generate, confirm, or modify instructions for the patient. Patient-doctor interaction, while not required for diagnostic and treatment, if used, may occur in person, real-time via an audio/video application, or by any other means of communication.

In embodiments, automated diagnostic and treatment system **102** may utilize images generated from a diagnostic examination of mouth, throat, eyes, ears, skin, extremities, surface abnormalities, internal imaging sources, and other suitable images and/or audio data generated from diagnostic examination of heart, lungs, abdomen, chest, joint motion, voice, and any other audio data sources. Automated diagnostic and treatment system **102** may further utilize patient lab tests, medical images, or any other medical data. In embodiments, automated diagnostic and treatment system **102** enables medical examination of the patient, for example, using medical devices, e.g., ultrasound, in medical instrument equipment **108** to detect sprains, contusions, or fractures, and automatically provide diagnostic recommendations regarding a medical condition of the patient.

In embodiments, diagnosis comprises the use of medical database decision vectors that are at least partially based on the patient's self-measured (or assistant-measured) vitals or other measured medical data, the accuracy score of a measurement dataset, a usage accuracy score of a sensor attached to medical instrument equipment **108,** a regional illness trend, and information used in generally accepted medical knowledge evaluations steps. The decision vectors and associated algorithms, which may be installed in automated diagnostic and treatment system **102,** may utilize one or more-dimensional data, patient history, patient questionnaire feedback, and pattern recognition or pattern matching for classification using images and audio data. In embodiments, a medical device usage accuracy score generator (not shown) may be implemented within automated diagnostic and treatment system **102** and may utilize an error vector of any device in medical instrument equipment or attached sensors **108** to create the device usage accuracy score and utilize the actual patient-measured device data to create the measurement data accuracy score.

In embodiments, automated diagnostic and treatment system **102** outputs diagnosis and/or treatment information that may be communicated to the patient, for example, electronically or in person by a medical professional, e.g., a treatment guideline that may include a prescription for a medication. In embodiments, prescriptions may be communicated directly to a pharmacy for pick-up or automated home delivery.

In embodiments, automated diagnostic and treatment system **102** may generate an overall health risk profile of the patient and recommend steps to reduce the risk of overlooking potentially dangerous conditions or guide the patient to a nearby facility that can treat the potentially dangerous condition. The health risk profile may assist a treating doctor in fulfilling duties to the patient, for example, to carefully review and evaluate the patient and, if deemed necessary, refer the patient to a specialist, initiate further testing, etc. The health risk profile advantageously reduces the potential for negligence and, thus, medical malpractice.

Automated diagnostic and treatment system **102,** in embodiments, comprises a payment feature that uses patient identification information to access a database to, e.g., determine whether a patient has previously arranged a method of payment, and if the database does not indicate a previously arranged method of payment, automated diagnostic and treatment system **102** may prompt the patient to enter payment information, such as insurance, bank, or credit card information. Automated diagnostic and treatment system **102** may determine whether payment information is valid and automatically obtain an authorization from the insurance, EHR system, and/or the card issuer for payment for a certain amount for services rendered by the doctor. An invoice may be electronically presented to the patient, e.g., upon completion of a consultation, such that the patient can authorize payment of the invoice, e.g., via an electronic signature.

In embodiments, patient database **103** (e.g., a secured cloud-based database) may comprise a security interface (not shown) that allows secure access to a patient database, for example, by using patient identification information to obtain the patient's medical history. The interface may utilize biometric, bar code, or other electronically security methods. In embodiments, medical instrument equipment **108** uses unique identifiers that are used as a control tool for measurement data. Database **103** may be a repository for any type of data created, modified, or received by diagnostic system **100,** such as generated diagnostic information, information received from patient's wearable electronic devices, remote video/audio data and instructions, e.g., instructions received from a remote location or from the application.

In embodiments, fields in the patient's electronic health care record (EHR) are automatically populated based on one or more of questions asked by diagnostic system **100,** measurements taken by the patient/system **100,** diagnosis and treatment codes generated by system **100,** one or more trust scores, and imported patient health care data from one or more sources, such as an existing health care database. It is understood the format of imported patient health care data may be converted to be compatible with the EHR format of system **100.** Conversely, exported patient health care data may be converted, e.g., to be compatible with an external EHR database.

In addition, patient-related data documented by system **100** provide support for the code decision for the level of exam a doctor performs. Currently, for billing and reimbursement purposes, doctors have to choose one of any identified codes (e.g., ICD10 currently holds approximately 97,000 medical codes) to identify an illness and provide an additional code that identifies the level of physical exam/diagnosis performed on the patient (e.g., full body physical exam) based on an illness identified by the doctor.

In embodiments, patient answers are used to suggest to the doctor a level of exam that is supported by the identified illness, e.g., to ensure that the doctor does not perform unnecessary in-depth exams for minor illnesses or a treatment that may not be covered by the patient's insurance.

In embodiments, upon identifying a diagnosis, system **100** generates one or more recommendations/suggestions/options for a particular treatment. In embodiments, one or more treatment plans are generated that the doctor may discuss with the patient and decide on a suitable treatment. For example, one treatment plan may be tailored purely for effectiveness, another one may consider the cost of drugs. In embodiments, system **100** may generate a prescription or lab test request and consider factors, such as recent research results, available drugs and possible drug interactions, the patient's medical history, traits of the patient, family history, and any other factors that may affect treatment when providing treatment information. In embodiments, diagnosis and treatment databases may be continuously updated, e.g., by health care professionals, so that an optimal treatment may be administered to a particular patient, e.g., a patient identified as member of a certain risk group.

It is noted that sensors and measurement techniques may be advantageously combined to perform multiple functions using a reduced number of sensors. For example, an optical sensor may be used as a thermal sensor by utilizing IR technology to measure body temperature. It is further noted that some or all data collected by system **100** may be processed and analyzed directly within automated diagnostic and treatment system **102** or transmitted to an external reading device (not shown in **FIG. 1****)** for further processing and analysis, e.g., to enable additional diagnostics.

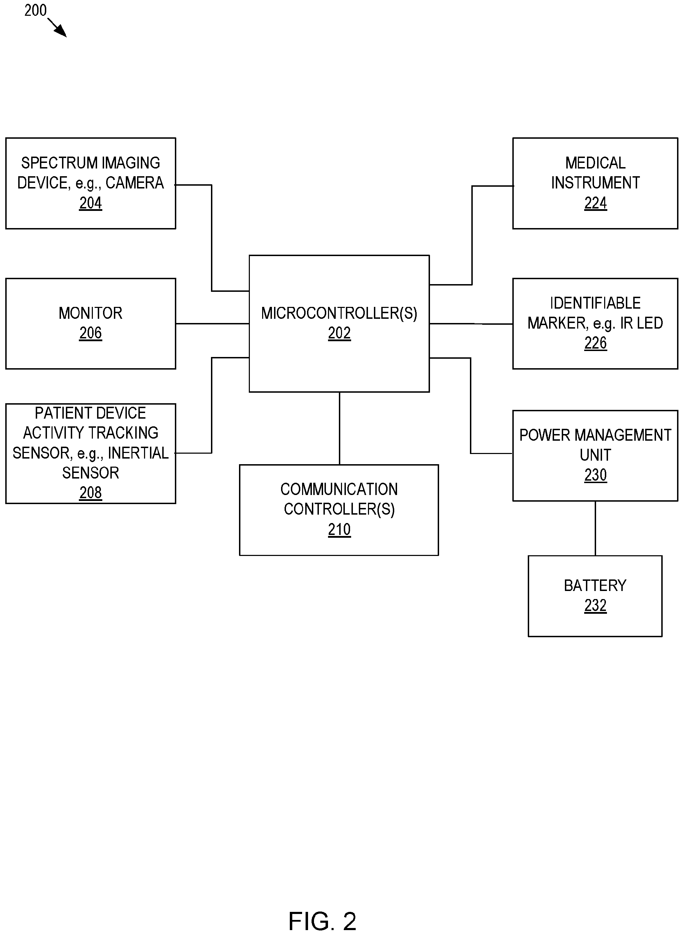

**FIG. 2** illustrates an exemplary patient diagnostic measurement system according to embodiments of the present disclosure. As depicted, patient diagnostic measurement system **200** comprises microcontroller **202,** spectrum imaging device, e.g., camera **204,** monitor **206,** patient-medical equipment activity tracking sensors, e.g., inertial sensor **208,** communications controller **210,** medical instruments **224,** identifiable marker, e.g., IR LED **226,** power management unit **230,** and battery **232.** Each component may be coupled directly or indirectly by electrical wiring, wirelessly, or optically to any other component in system **200.**

Medical instrument **224** comprises one or more devices that are capable of measuring physical and non-physical characteristics of a patient that, in embodiments, may be customized, e.g., according to varying anatomies among patients, irregularities on a patient's skin, and the like. In embodiments, medical instrument **224** is a combination of diagnostic medical devices that generate diagnostic data based on patient characteristics. Exemplary diagnostic medical devices are heart rate sensors, otoscopes, digital stethoscopes, in-ear thermometers, blood oxygen sensors, high-definition cameras, spirometers, blood pressure meters, respiration sensors, skin resistance sensors, glucometers, ultrasound devices, electrocardiographic sensors, body fluid sample collectors, eye slit lamps, weight scales, and any devices known in the art that may aid in performing a medical diagnosis. In embodiments, patient characteristics and vital signs data may be received from and/or compared against wearable or implantable monitoring devices that gather sample data, e.g., a fitness device that monitors physical activity.

One or more medical instruments **224** may be removably attachable directly to a patient's body, e.g., torso, via patches or electrodes that may use adhesion to provide good physical or electrical contact. In embodiments, medical instruments **224,** e.g., a contact-less thermometer, may perform contact-less measurements some distance away from the patient's body.

In embodiments, microcontroller **202** may be a secure microcontroller that securely communicates information in encrypted form to ensure privacy and the authenticity of measured data and activity sensor and patient-equipment proximity information and other information in patient diagnostic measurement system **200.** This may be accomplished by taking advantage of security features embedded in hardware of microcontroller **202** and/or software that enables security features during transit and storage of sensitive data. Each device in patient diagnostic measurement system **200** may have keys that handshake to perform authentication operations on a regular basis.

Spectrum imaging device camera **204** is any audio/video device that may capture patient images and sound at any frequency or image type. Monitor **206** is any screen or display device that may be coupled to camera, sensors and/or any part of system **200.** Patient-equipment activity tracking inertial sensor **208** is any single or multi-dimensional sensor, such as an accelerometer, a multi-axis gyroscope, pressure sensor, and a magnetometer capable of providing position, motion, pressure on medical equipment or orientation data based on patient interaction. Patient-equipment activity tracking inertial sensor **208** may be attached to (removably or permanently) or embedded into medical instrument **224.** Identifiable marker IR LED **226** represents any device, heat source, reflective material, proximity beacon, altimeter, etc., that may be used by microcontroller **202** as an identifiable marker. Like patient-equipment activity tracking inertial sensor **208,** identifiable marker IR LED **226** may be reattacheable to or embedded into medical instrument **224.**

In embodiments, communication controller **210** is a wireless communications controller attached either permanently or temporarily to medical instrument **224** or the patient's body to establish a bi-directional wireless communications link and transmit data, e.g., between sensors and microcontroller **202** using any wireless communication protocol known in the art, such as Bluetooth Low Energy, e.g., via an embedded antenna circuit that wirelessly communicates the data. One of ordinary skill in the art will appreciate that electromagnetic fields generated by such antenna circuit may be of any suitable type. In case of an RF field, the operating frequency may be located in the ISM frequency band, e.g., 13.56 MHz. In embodiments, data received by wireless communications controller **210** may be forwarded to a host device (not shown) that may run a software application.

In embodiments, power management unit **230** is coupled to microcontroller **202** to provide energy to, e.g., microcontroller **202** and communication controller **210.** Battery **232** may be a back-up battery for power management unit **230** or a battery in any one of the devices in patient diagnostic measurement system **200.** One of ordinary skill in the art will appreciate that one or more devices in system **200** may be operated from the same power source (e.g., battery **232)** and perform more than one function at the same or different times. A person of skill in the art will also appreciate that one or more components, e.g., sensors **208, 226,** may be integrated on a single chip/system, and that additional electronics, such as filtering elements, etc., may be implemented to support the functions of medical instrument equipment measurement or usage monitoring and tracking system **200** according to the objectives of the invention.

In operation, a patient may use medical instrument **224** to gather patient data based on physical and non-physical patient characteristics, e.g., vital signs data, images, sounds, and other information useful in the monitoring and diagnosis of a health-related condition. The patient data is processed by microcontroller **202** and may be stored in a database (not shown). In embodiments, the patient data may be used to establish baseline data for a patient health profile against which subsequent patient data may be compared.

In embodiments, patient data may be used to create, modify, or update EHR data. Gathered medical instrument equipment data, along with any other patient and sensor data, may be processed directly by patient diagnostic measurement system **200** or communicated to a remote location for analysis, e.g., to diagnose existing and expected health conditions to benefit from early detection and prevention of acute conditions or aid in the development of novel medical diagnostic methods.

In embodiments, medical instrument **224** is coupled to a number of sensors, such as patient-equipment tracking inertial sensor **208** and/or identifiable marker IR LED **226,** that may monitor a position/orientation of medical instrument **224** relative to the patient's body when a medical equipment measurement is taken. In embodiments, sensor data generated by sensor **208, 226** or other sensors may be used in connection with, e.g., data generated by spectrum imaging device camera **204,** proximity sensors, transmitters, bolometers, or receivers to provide feedback to the patient to aid the patient in properly aligning medical instrument **224** relative to the patient's body part of interest when performing a diagnostic measurement. A person skilled in the art will appreciate that not all sensors **208, 226,** beacon, pressure, altimeter, etc., need to operate at all times. Any number of sensors may be partially or completely disabled, e.g., to conserve energy.

In embodiments, the sensor emitter comprises a light signal emitted by IR LED **226** or any other identifiable marker that may be used as a reference signal. In embodiments, the reference signal may be used to identify a location, e.g., within an image and based on a characteristic that distinguishes the reference from other parts of the image. In embodiments, the reference signal is representative of a difference between the position of medical instrument **224** and a preferred location relative to a patient's body. In embodiments, spectrum imaging device camera **204** displays, e.g., via monitor **206,** the position of medical instrument **224** and the reference signal at the preferred location so as to allow the patient to determine the position of medical instrument **224** and adjust the position relative to the preferred location, displayed by spectrum imaging device camera **204.**

Spectrum imaging device camera **204,** proximity sensor, transmitter, receiver, bolometer, or any other suitable device may be used to locate or track the reference signal, e.g., within the image, relative to a body part of the patient. In embodiments, this may be accomplished by using an overlay method that overlays an image of a body part of the patient against an ideal model of device usage to enable real-time feedback for the patient. The reference signal along with signals from other sensors, e.g., patient-equipment activity inertial sensor **208,** may be used to identify a position, location, angle, orientation, or usage associated with medical instrument **224** to monitor and guide a patient's placement of medical instrument **224** at a target location and accurately activate a device for measurement.

In embodiments, e.g., upon receipt of a request signal, microcontroller **202** activates one or more medical instruments **224** to perform measurements and sends data related to the measurement back to microcontroller **202.** The measured data and other data associated with a physical condition may be automatically recorded and a usage accuracy of medical instrument **224** may be monitored.

In embodiments, microcontroller **202** uses an image in any spectrum, motion signal and/or an orientation signal by patient-equipment activity inertial sensor **208** to compensate or correct the vital signs data output by medical instrument **224.** Data compensation or correction may comprise filtering out certain data as likely being corrupted by parasitic effects and erroneous readings that result from medical instrument **224** being exposed to unwanted movements caused by perturbations or, e.g., the effect of movements of the patient's target measurement body part.

In embodiments, signals from two or more medical instruments **224,** or from medical instrument **224** and patient-activity activity system inertial sensor **208,** are combined, for example, to reduce signal latency and increase correlation between signals to further improve the ability of vital signs measurement system **200** to reject motion artifacts to remove false readings and, therefore, enable a more accurate interpretation of the measured vital signs data.

In embodiments, spectrum imaging device camera **204** displays actual or simulated images and videos of the patient and medical instrument **224** to assist the patient in locating a desired position for medical instrument **224** when performing the measurement so as to increase measurement accuracy. Spectrum imaging device camera **204** may use image or facial recognition software to identify and display eyes, mouth, nose, ears, torso, or any other part of the patient's body as reference.

In embodiments, vital signs measurement system **200** uses machine vision software that analyzes measured image data and compares image features to features in a database, e.g., to detect an incomplete image for a target body part, to monitor the accuracy of a measurement and determine a corresponding score. In embodiments, if the score falls below a certain threshold system **200** may provide detailed guidance for improving measurement accuracy or to receive a more complete image, e.g., by providing instructions on how to change an angle or depth of an otoscope relative to the patient's ear.

In embodiments, the machine vision software may use an overlay method to mimic a patient's posture/movements to provide detailed and interactive instructions, e.g., by displaying a character, image of the patient, graphic, or avatar on monitor **206** to provide feedback to the patient. The instructions, image, or avatar may start or stop and decide what help instruction to display based on the type of medical instrument **224,** the data from spectrum imaging device camera **204,** patient-equipment activity sensors inertial sensors **208,** bolometer, transmitter and receiver, and/or identifiable marker IR LED **226** (an image, a measured position or angle, etc.), and a comparison of the data to idealized data. This further aids the patient in correctly positioning and operating medical instrument **224** relative to the patient's body, ensures a high level of accuracy when operating medical instrument **224,** and solves potential issues that the patient may encounter when using medical instrument **224.**

In embodiments, instructions may be provided via monitor **206** and describe in audio/visual format and in any desired level of detail, how to use medical instrument **224** to perform a diagnostic test or measurement, e.g., how to take temperature, so as to enable patients to perform measurements of clinical-grade accuracy. In embodiments, each sensor **208, 226,** e.g., proximity, bolometer, transmitter/receiver may be associated with a device usage accuracy score. A device usage accuracy score generator (not shown), which may be implemented in microcontroller **202,** may use the sensor data to generate a medical instrument usage accuracy score that is representative of the reliability of medical instrument **224** measurement on the patient. In embodiments, the score may be based on a difference between an actual position of medical instrument **224** and a preferred position. In addition, the score may be based on detecting a motion, e.g., during a measurement. In embodiments, in response to determining that the accuracy score falls below a threshold, a repeat measurement or device usage assistance may be requested. In embodiments, the device usage accuracy score is derived from an error vector generated for one or more sensors **208, 226.** The resulting device usage accuracy score may be used when generating or evaluating medical diagnosis data.

In embodiments, microcontroller **202** analyzes the patient measured medical instrument data to generate a trust score indicative of the acceptable range of the medical instrument. For example, by comparing the medical instrument measurement data against reference measurement data or reference measurement data that would be expected from medical instrument **224.** As with device usage accuracy score, the trust score may be used when generating or evaluating a medical diagnosis data.

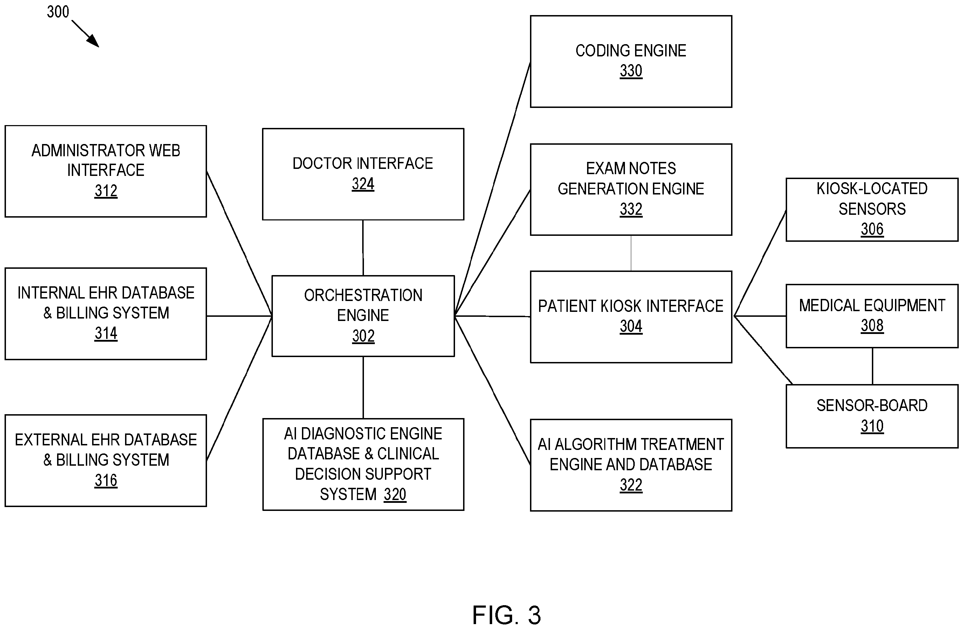

**FIG. 3** illustrates an exemplary medical data system according to embodiments of the present disclosure. Medical data system **300** comprises main orchestration engine **302,** patient kiosk interface **304,** doctor interface **324,** kiosk-located sensors **306,** medical equipment **308,** which may be coupled to sensor-board **310,** administrator web interface **312,** internal EHR database and billing system **314,** external EHR database and billing system **316,** Artificial Intelligence (AI) diagnostic engine and clinical decision support system **320,** AI treatment engine and database **322,** coding engine **330,** and exam notes generation engine **332.**

In operation, patient kiosk interface **304,** which may be a touch, voice, or text interface that is implemented into a tablet, computer, mobile device, or any other electronic device, receives patient-identifiable information, e.g., via a terminal or kiosk.

In embodiments, the patient-identifiable information and/or matching patient-identifiable information may be retrieved from internal EHR database and billing system **314** or searched for and downloaded from external EHR database **316,** which may be a public or private database such as a proprietary EHR database. In embodiments, if internal EHR database **314** and external EHR database **316** comprise no record for a patient, a new patient record may be created in internal EHR database and billing system **314.** In embodiments, internal EHR database and billing system **314** is populated based on external EHR database **316.**

In embodiments, data generated by medical equipment **308** is adjusted using measurement accuracy scores that are based on a position accuracy or reliability associated with using medical equipment **308,** e.g., by applying weights to the diagnostic medical information so as to correct for systemic equipment errors or equipment usage errors. In embodiments, generating the diagnostic data comprises adjusting diagnostic probabilities based on the device accuracy scores or a patient trust score associated with a patient. The patient trust score may be based on a patient response or the relative accuracy of responses by other patients. Orchestration engine **302** may record a patient's interactions with patient-kiosk interface **304** and other actions by a patient in diagnostic EHR database and billing system **314** or external EHR database and billing system **316.**

In embodiments, orchestration engine **302** coordinates gathering data from the AI diagnostic engine and clinical decision support system **320** which links gathered data to symptoms, diagnostics, and AI treatment engine and database **322** to automatically generate codes for tracking, diagnostic, follow-up, and billing purposes. Codes may comprise medical examination codes, treatment codes, procedural codes (e.g., numbers or alphanumeric codes that may be used to identify specific health interventions taken by medical professionals, such as ICPM, ICHI,and other codes that may describe a level of medical exam), lab codes, medical image codes, billing codes, pharmaceutical codes (e.g., ATC, NDC, and other codes that identify medications), and topographical codes that indicate a specific location in the body, e.g., ICD-O or SNOMED.

In embodiments, system **300** may comprise a diagnostic generation engine (not shown in FIG. 3) that may, for example, be implemented into coding engine **330.** The diagnostic generation engine may generate diagnostic codes e.g., ICD-9-CM, ICD-10, which may be used to identify diseases, disorders, and symptoms. In embodiments, diagnostic codes maybe used to determine morbidity and mortality.

In embodiments, generated codes may be used when generating a visit report and to update internal EHR database and billing system **314** and/or external EHR database and billing system **316.** In embodiments, the visit report is exported, e.g., by using a HIPAA compliant method, to external EHR database and billing system **316.** It is understood that while generated codes may follow any type of coding system and guidelines, rules, or regulations (e.g., AMA billing guidelines) specific to health care, other codes may equally be generated.

In embodiments, exam notes generation engine **332** aids a doctor in writing a report, such as a medical examination notes that may be used in a medical evaluation report. In embodiments, exam notes generation engine **332,** for example in concert with AI diagnostic engine and clinical decision support system **320** and/or AI treatment engine and database **322,** generates a text description of exam associated with the patient's use of system **300,** patient-doctor interaction, and diagnosis or treatment decisions resulting therefrom. Exam notes may comprise any type of information related to the patient, items examined by system **300** or a doctor, diagnosis justification, treatment option justification, and other conclusions. As an example, exam notes generation engine **332** may at least partially populate a text with notes related to patient information, diagnostic medical information, and other diagnosis or treatment options and a degree of their relevance (e.g., numerical/probabilistic data that supports a justification why a specific illness or treatment option was not selected).

In embodiments, notes comprise patient-related data such as patient interaction information that was used to make a recommendation regarding a particular diagnosis or treatment. It is understood that the amount and detail of notes may reflect the closeness of a decision and the severity of an illness. Doctor notes for treatment may comprise questions that a patient was asked regarding treatment and reasons for choosing a particular treatment and not choosing alternative treatments provided by AI treatment engine and database **322.** In embodiments, provider notes may be included in a patient visit report and also be exported to populate an internal or external EHR database and billing system.

In embodiments, orchestration engine **302** forwards a request for patient-related data to patient kiosk interface **304** to solicit patient input either directly from a patient, assistant, or medical professional in the form of answers to questions or from a medical equipment measurement (e.g., vital signs). The patient-related data may be received via secure communication and stored in internal EHR database and billing system **314.** It is understood that patient input may include confirmation information, e.g., for a returning patient.

In embodiments, based on an AI diagnostic algorithm, orchestration engine **302** may identify potential illnesses and request additional medical and/or non-medical (e.g., sensor) data that may be used to narrow down the number of potential illnesses and generate diagnostic probability results.

In embodiments, the diagnostic result is generated by using, for example, data from kiosk-located sensors **306,** medical equipment **308,** sensor board **310,** patient history, demographics, and preexisting conditions stored in either the internal EHR **314** or external EHR database **316.** In embodiments, orchestration engine **302,** AI diagnostic engine and clinical decision support system **320,** or a dedicated exam level generation engine (not shown in FIG 3.) suggests a level of exam in association with illness risk profile and commonly accepted physical exam guidelines. Exam level, actual exams performed, illness probability, and other information may be used for provider interface checklists that meet regulatory requirements and to generate billing codes.

In embodiments, AI diagnostic engine and clinical decision support system **320,** may generate a criticality score representative of probability that a particular patient will suffer a negative health effect for a period of time from non-treatment may be assigned to an illness or a combination of illnesses as related to a specific patient situation. Criticality scores may be generated by taking into account patients' demographic, medical history, and any other patient-related information. Criticality scores may be correlated to each other and normalized and, in embodiments, may be used when calculating a malpractice score for the patient. A criticality score of 1, for example, may indicate that no long-lasting negative health effect is expected from an identified illness, while a criticality score of 10 indicates that that the patient may prematurely die if the patient in fact has that illness, but the illness remains untreated.

As an example, a mid-aged patient of general good health and having weight, blood pressure, and BMI data indicating a normal or healthy range is not likely to suffer long-lasting negative health effects from non-treatment of a common cold. As a result, a criticality score of 1 may be assigned to the common cold for this patient. Similarly, the previously referred to patient is likely to suffer moderately negative health effects from non-treatment of strep throat, such that a criticality score of 4 may be assigned for strep throat. For the same patient, the identified illness of pneumonia may be assigned a criticality score of 6, and for lung cancer, the criticality score may be 9.

In embodiments, AI treatment engine and database **322** receives from AI diagnostic engine and clinical decision support system **320** diagnosis and patient-related data, e.g., in the form of a list of potential illnesses and their probabilities together with other information about a patient. Based on the diagnosis and patient-related data, AI treatment engine and database **322** may determine treatment recommendations/suggestions/options for each of the potential illness. Treatment options may comprise automatically selected medicines, instructions to the patient regarding a level of physical activities, nutrition, or other instruction, and exam requests, such as suggested patient treatment actions, lab tests, and imaging requests that may be shared with a doctor.

In embodiments, AI treatment engine and database **322** generates for a patient a customized treatment plan, for example, based on an analysis of likelihoods of success for each of a set of treatment options, taking into consideration factors such as possible drug interactions and side-effects (e.g., whether the patient is allergic to certain medicines), patient demographics, and the like. In embodiments, the treatment plan comprises instructions to the patient, requests for medical images and lab reports, and suggestion regarding medication. In embodiments, treatment options are ranked according to the likelihood of success for a particular patient.

In embodiments, AI treatment engine and database **322** is communicatively coupled to a provider application (e.g., on a tablet) to exchange treatment information with a doctor. The provider application may be used to provide the doctor with additional details on diagnosis and treatment, such as information regarding how a probability for each illness or condition is calculated and based on which symptom(s) or why certain medication was chosen as the best treatment option.

For example, once a doctor accepts a patient, a monitor may display the output of Al diagnostic engine and clinical decision support system **320** as showing a 60% probability that the patient has the common cold, 15% probability that the patient has strep throat, 15% probability that the patient has an ear infection, 5% probability that the patient has pneumonia, and a 5% probability that the patient has lung cancer. In embodiments, the probabilities are normalized, such that their sum equals 100% or some other measure that relates to the provider.

In embodiments, the doctor may select items on a monitor to access additional details such as images, measurement data, and any other patient-related information, e.g., to evaluate a treatment plan proposed by AI treatment engine and database **322.**

In embodiments, AI treatment engine and database **322** may determine the risk of false negative for each illness (e.g., strep, pneumonia, and lung cancer), even of a doctor determined that the patient has only one illness, and initiate test/lab requests, e.g., a throat swab for strep, and an x-ray for pneumonia/lung cancer that may be ordered automatically or with doctor approval (e.g., per AMA guidelines).

In embodiments, AI treatment engine and database **322** combines gathered data with symptom, diagnostic, and treatment information and generates any type of diagnostic, physical exam level, and treatment codes, for example, for billing purposes or to satisfy regulatory requirements and/or physician guidelines, billing, and continued treatment purposes. In embodiments, treatment codes are based on drug information, a patient preference, a patient medical history, and the like.

In embodiments, codes, doctor notes, and a list of additional exam requirements may be included in a visit report and used to automatically update internal EHR database and billing system **314** or external EHR database and billing system **316.** The visit report may then be exported, e.g., by a HIPAA compliant method, to external EHR database and billing system **316.**

In embodiments, AI treatment engine and database **322** estimates when a particular treatment is predicted to show health improvements for the patient. For example, if a predicted time period during which a patient diagnosed with strep throat and receiving antibiotics should start feeling better is three days, then, on the third day, medical data system **300** may initiate a notice to the patient to solicit patient feedback to determine whether the patient's health condition, in fact, has improved as predicted.

It is understood that patient may be asked to provide feedback at any time and that feedback may comprise any type of inquiries related to the patient's condition. In embodiments, the inquiry comprises questions regarding the patient's perceived accuracy of the diagnosis and/or treatment. In embodiments, if the patient does not affirm the prediction, e.g., because the symptoms are worsening or the diagnoses was incorrect, the patient may be granted continued access to system **300** for a renewed diagnosis.

In embodiments, any part of the information gathered by medical data system **300** may be utilized to improve machine learning processes of Al diagnostic engine and clinical decision support system **320** and AI treatment engine and database **322** and, thus, improve diagnoses and treatment capabilities of system **300.**

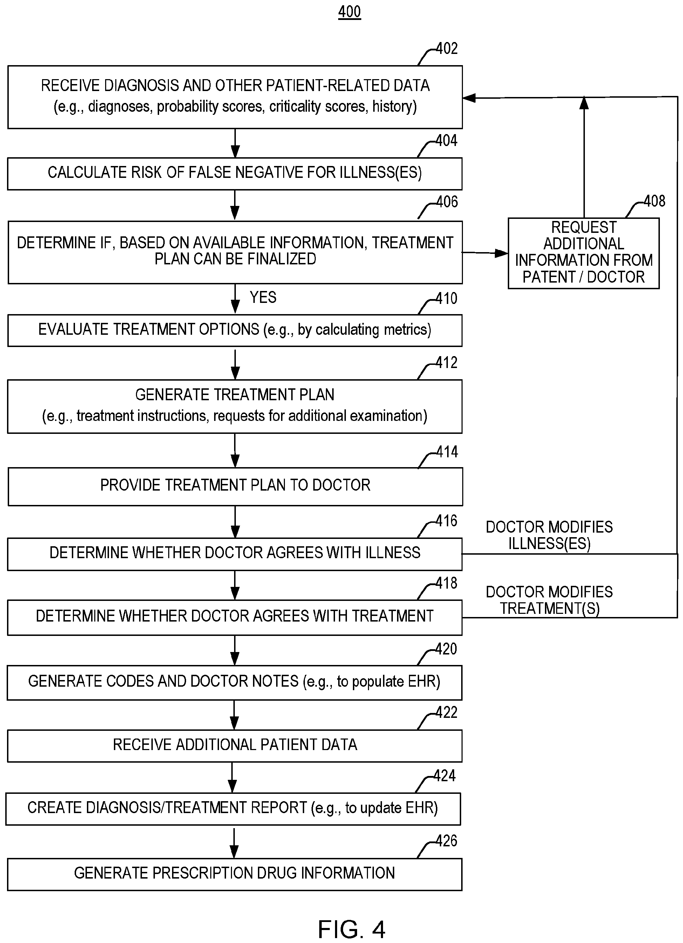

**FIG. 4** is a flowchart of an illustrative process for generating patient treatment information according to embodiments of the present disclosure. Process **400** begins at step **402** when a diagnosis is received, e.g., from a diagnostic engine as described herein. The diagnostic engine may output a diagnosis for a patient based on computer-generated or computer-aided diagnoses. In embodiments, the diagnosis is generated by diagnostic engine and received at a treatment engine. The treatment engine may further receive patient-related data, e.g., patient medical history retrieved from a public EHR database, possible allergies to medicine, previous visit information, current medicine usage, etc.

In embodiments, the diagnosis may comprise one or more potential illnesses or symptoms identified from a set of potential illnesses and symptoms. Each individual illness may be associated with a probability. In addition, each illness or combination of illnesses may be assigned a criticality score described above.

At step **404,** the risk of a false negative may be determined for each illness.

At step **406,** based on the computer-aided diagnosis and other patient-related data, for each potential illness, it is determined whether a treatment plan can be finalized based on available information. Treatment plans may comprise one or more of prescription drugs, lab work, imaging, medical tests, and doctor requests to the patient.

If at step **406** it is determined that a treatment plan cannot be finalized, then process **400** proceeds with step **408,** by requesting, e.g., at a kiosk or doctor station, additional information from the patient/doctor, such as known allergies, past medical history, or medicine usage. Process **400** may then resume with any of steps **402** and **406,** until it is determined that a treatment plan can be finalized.

At step **410,** each treatment option may be evaluated-individually or in combination, e.g., by assigning a likelihood of success to each treatment option that may be considered by the patient/doctor. In embodiments, additional or other metrics, e.g., cost of treatment, may be used to evaluate treatment options, again, individually and in combination.

At step **412,** based on the evaluation, a treatment plan is generated for the patient. In embodiments, the treatment plan may comprise patient treatment instructions (e.g., medication and dosages) and/or requests for additional examination (e.g., medical images and lab reports) and/or other suggestions (e.g., get more rest, eat healthier, etc.).

At step **414,** the treatment plan is communicated to a doctor, e.g., via a provider application, for confirmation.

At step **416,** it is determined whether, after an appropriate review, diagnoses/illnesses are acceptable to a doctor. If not, for example, because a doctor decides that the patient has a subset of the displayed illnesses or an entirely different illness, the doctor may choose to delete, modify, or add illnesses at this point, and process **400** may continue with step **402** after accepting the doctor's input.

At step **418,** it is determined whether a treatment for illness(es) is acceptable to the doctor. If not, the doctor may choose to delete, modify, or add a treatment, and process **400** may continue with step **402,** e.g., in order to reduce the potential for conflicts with other patient-related information. In embodiments, if a conflict related to the treatment suggested by the doctor is found, information about that conflict may be conveyed to the doctor, e.g., to review why a particular treatment was suggested for an illness or group of illnesses.

At step **420,** treatment codes and doctor notes may be generated, e.g., to populate a patient EHR.

At step **422,** additional patient-related data, such as patient feedback, test/lab data, may be received.

At step **424,** a diagnosis and/or treatment report may be created, and the results may be used to update the external EHR database.

At step **426,** prescription drug information may be electronically generated e.g., per an electronic request that is automatically created, and communicated to an appropriate person or entity to facilitate fulfillment.

One skilled in the art will recognize that: (1) certain steps may optionally be performed; (2) steps may not be limited to the specific order set forth herein; and (3) certain steps may be performed in different orders; and (4) certain steps may be done concurrently.

In embodiments, one or more computing systems, such as mobile/tablet/computer or the automated diagnostic system, may be configured to perform one or more of the methods, functions, and/or operations presented herein. Systems that implement at least one or more of the methods, functions, and/or operations described herein may comprise an application or applications operating on at least one computing system. The computing system may comprise one or more computers and one or more databases. The computer system may be a single system, a distributed system, a cloud-based computer system, or a combination thereof.

It shall be noted that the present disclosure may be implemented in any instruction-execution/computing device or system capable of processing data, including, without limitation phones, laptop computers, desktop computers, and servers. The present disclosure may also be implemented into other computing devices and systems. Furthermore, aspects of the present disclosure may be implemented in a wide variety of ways including software (including firmware), hardware, or combinations thereof. For example, the functions to practice various aspects of the present disclosure may be performed by components that are implemented in a wide variety of ways including discrete logic components, one or more application specific integrated circuits (ASICs), and/or program-controlled processors. It shall be noted that the manner in which these items are implemented is not critical to the present disclosure.

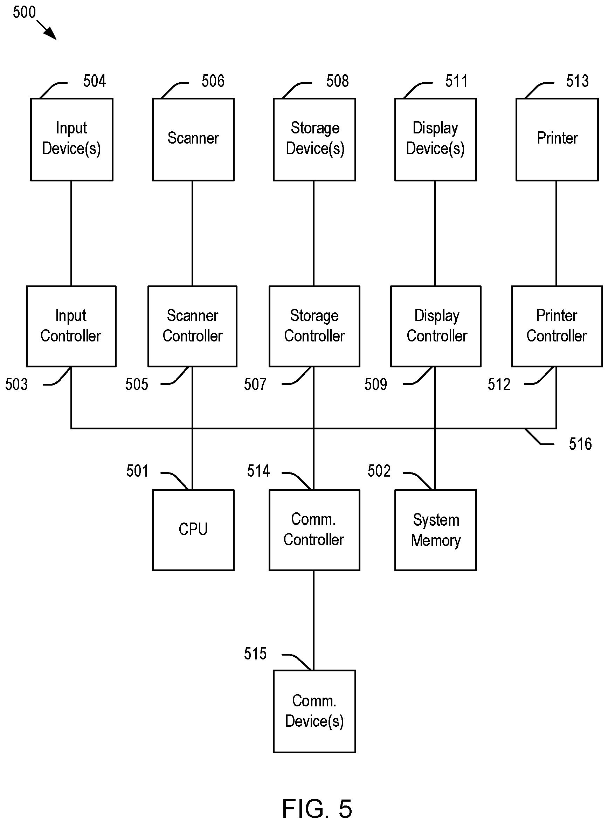

Having described the details of the disclosure, an exemplary system that may be used to implement one or more aspects of the present disclosure is described next with reference to **FIG. 5****.** Each of patient interface station **106** and automated diagnostic system **102** in FIG. 1 may comprise one or more components in the system **500.** As illustrated in **FIG. 5****,** system **500** includes a central processing unit (CPU) **501** that provides computing resources and controls the computer. CPU **501** may be implemented with a microprocessor or the like, and may also include a graphics processor and/or a floating point coprocessor for mathematical computations. System **500** may also include a system memory **502,** which may be in the form of random-access memory (RAM) and read-only memory (ROM).

A number of controllers and peripheral devices may also be provided, as shown in **FIG. 5****.** An input controller **503** represents an interface to various input device(s) **504,** such as a keyboard, mouse, or stylus. There may also be a scanner controller **505,** which communicates with a scanner **506.** System **500** may also include a storage controller **507** for interfacing with one or more storage devices **508** each of which includes a storage medium such as magnetic tape or disk, or an optical medium that might be used to record programs of instructions for operating systems, utilities and applications which may include embodiments of programs that implement various aspects of the present disclosure. Storage device(s) **508** may also be used to store processed data or data to be processed in accordance with the disclosure. System **500** may also include a display controller **509** for providing an interface to a display device **511,** which may be a cathode ray tube (CRT), a thin film transistor (TFT) display, or other type of display. System **500** may also include a printer controller **512** for communicating with a printer **55.** A communications controller **514** may interface with one or more communication devices **515,** which enables system **500** to connect to remote devices through any of a variety of networks including the Internet, an Ethernet cloud, an FCoE/DCB cloud, a local area network (LAN), a wide area network (WAN), a storage area network (SAN) or through any suitable electromagnetic carrier signals including infrared signals.

In the illustrated system, all major system components may connect to a bus **516,** which may represent more than one physical bus. However, various system components may or may not be in physical proximity to one another. For example, input data and/or output data may be remotely transmitted from one physical location to another. In addition, programs that implement various aspects of this disclosure may be accessed from a remote location (e.g., a server) over a network. Such data and/or programs may be conveyed through any of a variety of machine-readable medium including, but are not limited to: magnetic media such as hard disks, floppy disks, and magnetic tape; optical media such as CD-ROMs and holographic devices; magneto-optical media; and hardware devices that are specially configured to store or to store and execute program code, such as application specific integrated circuits (ASICs), programmable logic devices (PLDs), flash memory devices, and ROM and RAM devices.

Embodiments of the present disclosure may be encoded upon one or more non-transitory computer-readable media with instructions for one or more processors or processing units to cause steps to be performed. It shall be noted that the one or more non-transitory computer-readable media shall include volatile and non-volatile memory. It shall be noted that alternative implementations are possible, including a hardware implementation or a software/hardware implementation. Hardware-implemented functions may be realized using ASIC(s), programmable arrays, digital signal processing circuitry, or the like. Accordingly, the "means" terms in any claims are intended to cover both software and hardware implementations. Similarly, the term "computer-readable medium or media" as used herein includes software and/or hardware having a program of instructions embodied thereon, or a combination thereof. With these implementation alternatives in mind, it is to be understood that the figures and accompanying description provide the functional information one skilled in the art would require to write program code (i.e., software) and/or to fabricate circuits (i.e., hardware) to perform the processing required.

It shall be noted that embodiments of the present disclosure may further relate to computer products with a non-transitory, tangible computer-readable medium that have computer code thereon for performing various computer-implemented operations. The media and computer code may be those specially designed and constructed for the purposes of the present disclosure, or they may be of the kind known or available to those having skill in the relevant arts. Examples of tangible computer-readable media include, but are not limited to: magnetic media such as hard disks, floppy disks, and magnetic tape; optical media such as CD-ROMs and holographic devices; magneto-optical media; and hardware devices that are specially configured to store or to store and execute program code, such as application specific integrated circuits (ASICs), programmable logic devices (PLDs), flash memory devices, and ROM and RAM devices. Examples of computer code include machine code, such as produced by a compiler, and files containing higher level code that are executed by a computer using an interpreter. Embodiments of the present disclosure may be implemented in whole or in part as machine-executable instructions that may be in program modules that are executed by a processing device. Examples of program modules include libraries, programs, routines, objects, components, and data structures. In distributed computing environments, program modules may be physically located in settings that are local, remote, or both.

For purposes of this disclosure, an information handling system may include any instrumentality or aggregate of instrumentalities operable to compute, calculate, determine, classify, process, transmit, receive, retrieve, originate, switch, store, display, communicate, manifest, detect, record, reproduce, handle, or utilize any form of information, intelligence, or data for business, scientific, control, or other purposes. For example, an information handling system may be a personal computer (e.g., desktop or laptop), tablet computer, mobile device (e.g., personal digital assistant (PDA) or smart phone), server (e.g., blade server or rack server), a network storage device, or any other suitable device and may vary in size, shape, performance, functionality, and price. The information handling system may include random access memory (RAM), one or more processing resources such as a central processing unit (CPU) or hardware or software control logic, ROM, and/or other types of nonvolatile memory. Additional components of the information handling system may include one or more disk drives, one or more network ports for communicating with external devices as well as various input and output (I/O) devices, such as a keyboard, a mouse, touchscreen and/or a video display. The information handling system may also include one or more buses operable to transmit communications between the various hardware components.

One skilled in the art will recognize no computing system or programming language is critical to the practice of the present disclosure. One skilled in the art will also recognize that a number of the elements described above may be physically and/or functionally separated into sub-modules or combined together.

## Claims

1. A medical data system for generating patient treatment instructions, the system comprising:
a patient interface 106 304) with medical equipment coupled to a plurality of sensors, the interface configured to receive patient-related data as direct input from a patient and medical equipment measurements taken by the patient by using the medical equipment (308), and a signal from the plurality of sensors comprising a position and orientation of at least one medical instrument (224) of the medical equipment when the medical equipment measurements are taken,
wherein the plurality of sensors comprise an infrared light-emitting diode (226) and a patient-equipment tracking inertial sensor (208) attached to the medical instrument (224), wherein the interface comprises a spectrum imaging device camera (204) coupled to a monitor (206), wherein the spectrum imaging device camera (204) is configured to display via the monitor (206) the position of the medical instrument (224) and a reference signal formed by a light signal emitted by the infrared light-emitting diode (226) so as to allow the patient to determine the position of the medical instrument (224) and to adjust the position relative to a target location displayed by the spectrum imaging device camera (204),
wherein the interface is further configured to generate a medical instrument usage accuracy score representative of the reliability of the medical instrument measurement on the patient, the accuracy score based on the position of the medical instrument (224) and the target location, the interface further configured to determine if the accuracy score falls below a threshold and generate a request for a repeat measurement or a device usage assistance for positioning the medical instrument (224) in response, the interface further configured to generate a trust score indicative of an acceptable range of the medical instrument (224) by comparing the medical equipment measurements against reference measurement data that would be expected from the medical instrument (224);
a diagnostic engine utilizing a machine learning processes to calculate diagnostic data comprising probabilities associated with illnesses that have been identified from a set of potential illnesses, wherein the accuracy score and the trust score are used when calculating the diagnostic data;
a treatment engine coupled to the diagnostic engine, the treatment engine receives the diagnostic data from the diagnostic engine and performs the steps of:
determining a treatment option for one or more of the illnesses;
analyzing a likelihood of success for the treatment option;
based on the analysis, generating patient treatment instructions for one or more treatment options;
receiving patient feedback regarding the patient treatment instructions, the patient feedback comprising information on a patient condition and an accuracy of a treatment as perceived by the patient; and
providing the patient feedback to the diagnostic engine to improve the machine learning processes.

2. The medical data system according to claim 1, wherein the treatment engine assigns criticality scores to one or more of the illnesses.

3. The medical data system according to claim 1, wherein the diagnostic engine populates an electronic health care record (EHR).

4. The medical data system according to claim 3, further comprising a notes generation engine that generates exam notes to populate the EHR.

5. The medical data system according to claim 1, wherein the medical data system comprises a coding engine that, based on at least one of drug information, a patient preference, and a patient medical history, generates an output that comprises a treatment code.

6. The medical data system according to claim 5, wherein the treatment engine uses at least one of the treatment plan and the treatment code to update an EHR.

7. A method for generating patient treatment instructions, the method comprising:
- at a patient interface 106 304) with medical equipment coupled to a plurality of sensors,
receiving patient-related data as direct input from a patient and medical equipment measurements taken by the patient by using the medical equipment, and a signal from the plurality of sensors comprising a position and orientation of a medical instrument of the medical equipment when the medical equipment measurements are taken, the plurality of sensors comprising an infrared light-emitting diode (226) and a patient-equipment tracking inertial sensor (208) attached to the medical instrument (224) of the medical equipment, wherein the interface comprises a spectrum imaging device camera (204) coupled to a monitor (206), wherein the spectrum imaging device camera (204) displays via the monitor (206) the position of the medical instrument (224) and a reference signal formed by a light signal emitted by the infrared light-emitting diode (226) so as to allow the patient to determine the position of the medical instrument (224) and to adjust the position relative to a target location displayed by the spectrum imaging device camera (204),
wherein the interface generates a medical instrument usage accuracy score representative of the reliability of the medical instrument measurement on the patient, the accuracy score based on the position of the medical instrument (224) and the target location, wherein the interface determines if the accuracy score falls below a threshold and generates a request for a repeat measurement or a device usage assistance for positioning the medical instrument (224) in response, the interface further generating a trust score indicative of an acceptable range of the medical instrument (224) by comparing the medical equipment measurements against reference measurement data that would be expected from the medical instrument (224);
- at a diagnostic engine, utilizing a machine learning processes to calculate diagnostic data comprising probabilities associated with illnesses that have been identified from a set of potential illnesses, and using the accuracy score and the trust score when calculating the diagnostic data;
- at a treatment engine, receiving the diagnostic data from the diagnostic engine;
based on the diagnostic data, determining a treatment option for one or more of the illnesses;
analyzing a likelihood of success for the treatment option;
based on the analysis, generating patient treatment instructions for one or more treatment options;
receiving patient feedback regarding the patient treatment instructions, the patient feedback comprising information on a patient condition and an accuracy of a treatment as perceived by the patient; and
providing the patient feedback to the diagnostic engine to improve the machine learning processes.

8. The method according to claim 7, wherein the one or more of illnesses may be assigned criticality scores.

9. The method according to claim 7, further comprising receiving patient-related data from an electronic health care record (EHR).

10. The method according to claim 7, further comprising, based on the diagnostic data, generating an output that comprises a treatment code that is based on at least one of drug information, a patient preference, and a patient medical history.

11. The method according to claim 10, further comprising using at least one of the treatment instructions and the treatment code to update an EHR.

12. The method according to claim 10, further comprising generating exam notes to populate the EHR.

13. The method according to claim 7, further comprising recalculating the likelihood of success to adjust the treatment plan.

14. The method according to claim 7, wherein the patient treatment instructions comprise requests for additional examination.

15. The method according to claim 14, further comprising calculating a risk of a false negative for the one or more illnesses.

## Patentansprüche

1. Medizinisches Datensystem zum Erzeugen von Patientenbehandlungsanweisungen, wobei das System Folgendes umfasst:
eine Patientenschnittstelle (106, 304) mit medizinischen Geräten, die mit einer Vielzahl von Sensoren gekoppelt sind, wobei die Schnittstelle konfiguriert ist, um patientenbezogene Daten als direkte Eingabe von einem Patienten und Messungen von medizinischen Geräten (308), die von dem Patienten unter Verwendung der medizinischen Geräte vorgenommen werden, und ein Signal von der Vielzahl von Sensoren zu empfangen, das eine Position und Ausrichtung von mindestens einem medizinischen Instrument (224) der medizinischen Geräte umfasst, wenn die Messungen der medizinischen Geräte vorgenommen werden,
wobei die Vielzahl von Sensoren eine Infrarotlicht emittierende Diode (226) und einen an dem medizinischen Instrument (224) angebrachten Trägheitssensor (208) zur Verfolgung des Patientengeräts umfasst, wobei die Schnittstelle eine mit einem Bildschirm (206) gekoppelte Kamera (204) einer Spektralabbildungsvorrichtung umfasst, wobei die Kamera (204) der Spektralabbildungsvorrichtung konfiguriert ist, um die Position des medizinischen Instruments (224) und ein Referenzsignal, das durch ein von der Infrarotlicht emittierenden Diode (226) emittiertes Lichtsignal gebildet wird, über den Bildschirm (206) anzuzeigen, um so dem Patienten zu ermöglichen, die Position des medizinischen Instruments (224) zu bestimmen und die Position bezogen auf einen Zielort, der von der Kamera (204) der Spektralabbildungsvorrichtung angezeigt wird, anzupassen,
wobei die Schnittstelle ferner konfiguriert ist, um einen Verwendungsrichtigkeitswert des medizinischen Instruments, der für die Zuverlässigkeit der Messung des medizinischen Instruments am Patienten repräsentativ ist, zu erzeugen, wobei der Richtigkeitswert auf der Position des medizinischen Instrument (224) und dem Zielort basiert, wobei die Schnittstelle ferner konfiguriert ist, um zu bestimmen, ob der Richtigkeitswert unter einen Schwellenwert fällt, und als Reaktion eine Anforderung für eine Wiederholungsmessung oder eine Vorrichtungsnutzungsunterstützung zum Positionieren des medizinischen Instruments (224) zu erzeugen, wobei die Schnittstelle ferner konfiguriert ist, um einen Vertrauenswert zu erzeugen, der einen zulässigen Bereich des medizinischen Instruments (224) angibt, indem die Messungen der medizinischen Geräte mit Referenzmessdaten verglichen werden, die von dem medizinischen Instrument (224) zu erwarten wären;
eine Diagnosemaschine, die maschinelle Lernprozesse verwendet, um Diagnosedaten zu berechnen, die Wahrscheinlichkeiten umfassen, die mit Krankheiten verbunden sind, die aus einem Satz potenzieller Krankheiten identifiziert wurden, wobei der Richtigkeitswert und der Vertrauenswert bei der Berechnung der Diagnosedaten verwendet werden;
eine mit der Diagnosemaschine gekoppelte Behandlungsmaschine, wobei die Behandlungsmaschine die Diagnosedaten von der Diagnosemaschine empfängt und die folgenden Schritte durchführt: Bestimmen einer Behandlungsoption für eine oder mehrere der Krankheiten;
Analysieren der Erfolgswahrscheinlichkeit der Behandlungsoption;
Erzeugen von Patientenbehandlungsanweisungen für eine oder mehrere Behandlungsoptionen basierend auf der Analyse;
Empfangen von Patientenrückmeldungen bezüglich der Patientenbehandlungsanweisungen, wobei die Patientenrückmeldungen Informationen über einen Patientenzustand und eine von dem Patienten wahrgenommene Richtigkeit einer Behandlung umfassen; und
Bereitstellen der Patientenrückmeldung der Diagnosemaschine, um die maschinellen Lernprozesse zu verbessern.

2. Medizinisches Datensystem nach Anspruch 1, wobei die Behandlungsmaschine einer oder mehreren Krankheiten Kritikalitätswerte zuweist.

3. Medizinisches Datensystem nach Anspruch 1, wobei die Diagnosemaschine eine elektronische Patientenakte (ePA) befüllt.

4. Medizinisches Datensystem nach Anspruch 3, ferner umfassend eine Notizenerzeugungsmaschine, die Untersuchungsnotizen zum Befüllen der ePA erzeugt.

5. Medizinische Datensystem nach Anspruch 1, wobei das medizinische Datensystem eine Codiermaschine umfasst, die basierend auf mindestens einer Arzneimittelinformation, einer Patientenpräferenz und einer Krankengeschichte des Patienten eine Ausgabe erzeugt, die einen Behandlungscode umfasst.

6. Medizinisches Datensystem nach Anspruch 5, wobei die Behandlungsmaschine den Behandlungsplan und/oder den Behandlungscode verwendet, um eine ePA zu aktualisieren.

7. Verfahren zum Erzeugen von Patientenbehandlungsanweisungen, wobei das Verfahren umfasst:
- an einer Patientenschnittstelle (106, 304) mit medizinischen Geräten, die mit einer Vielzahl von Sensoren gekoppelt sind,
Empfangen von patientenbezogenen Daten als direkte Eingabe von einem Patienten und von Messungen von medizinischen Geräten, die von dem Patienten unter Verwendung der medizinischen Geräte vorgenommen werden, und eines Signals von der Vielzahl von Sensoren, das eine Position und Ausrichtung eines medizinischen Instruments der medizinischen Geräte umfasst, wenn die Messungen der medizinischen Geräte vorgenommen werden, wobei die Vielzahl von Sensoren eine Infrarotlicht emittierende Diode (226) und einen an dem medizinischen Instrument (224) angebrachten Trägheitssensor (208) zur Verfolgung des Patientengeräts der medizinischen Geräte umfassen, wobei die Schnittstelle eine mit einem Bildschirm (206) gekoppelte Kamera (204) einer Spektralabbildungsvorrichtung umfasst, wobei die Kamera (204) der Spektralabbildungsvorrichtung die Position des medizinischen Instruments (224) und ein Referenzsignal, das durch ein von der Infrarotlicht emittierenden Diode (226) emittiertes Lichtsignal gebildet wird, über den Bildschirm (206) anzeigt, um so dem Patienten zu ermöglichen, die Position des medizinischen Instruments (224) zu bestimmen und die Position bezogen auf einen Zielort, der von der Kamera (204) der Spektralabbildungsvorrichtung angezeigt wird, anzupassen, wobei die Schnittstelle ferner einen Verwendungsrichtigkeitswert des medizinischen Instruments, der für die Zuverlässigkeit der Messung des medizinischen Instruments am Patienten repräsentativ ist, erzeugt, wobei der Richtigkeitswert auf der Position des medizinischen Instrument (224) und dem Zielort basiert, wobei die Schnittstelle bestimmt, ob der Richtigkeitswert unter einen Schwellenwert fällt, und als Reaktion eine Anforderung für eine Wiederholungsmessung oder eine Vorrichtungsnutzungsunterstützung zum Positionieren des medizinischen Instruments (224) erzeugt, wobei die Schnittstelle ferner einen Vertrauenswert erzeugt, der einen zulässigen Bereich des medizinischen Instruments (224) angibt, indem die Messungen der medizinischen Geräte mit Referenzmessdaten verglichen werden, die von dem medizinischen Instrument (224) zu erwarten wären;
- an einer Diagnosemaschine, Verwenden der maschinellen Lernprozesse, um Diagnosedaten zu berechnen, die Wahrscheinlichkeiten umfassen, die mit Krankheiten verbunden sind, die aus einem Satz potenzieller Krankheiten identifiziert wurden, und Verwenden des Richtigkeitswerts und des Vertrauenswerts bei der Berechnung der Diagnosedaten;
- an einer Behandlungsmaschine, Empfangen der Diagnosedaten von der Diagnosemaschine;
Bestimmen einer Behandlungsoption für eine oder mehrere der Krankheiten basierend auf den Diagnosedaten;
Analysieren der Erfolgswahrscheinlichkeit der Behandlungsoption;
Erzeugen von Patientenbehandlungsanweisungen für eine oder mehrere Behandlungsoptionen basierend auf der Analyse;
Empfangen von Patientenrückmeldungen bezüglich der Patientenbehandlungsanweisungen, wobei die Patientenrückmeldungen Informationen über einen Patientenzustand und eine von dem Patienten wahrgenommene Richtigkeit einer Behandlung umfassen; und
Bereitstellen der Patientenrückmeldung der Diagnosemaschine, um die maschinellen Lernprozesse zu verbessern.

8. Verfahren nach Anspruch 7, wobei der einen oder mehreren Krankheiten Kritikalitätswerte zugeordnet werden können.

9. Verfahren nach Anspruch 7, ferner umfassend Empfangen von patientenbezogenen Daten aus einer elektronischen Patientenakte (ePA).

10. Verfahren nach Anspruch 7, ferner umfassend, basierend auf den Diagnosedaten, Erzeugen einer Ausgabe, die einen Behandlungscode umfasst, der auf mindestens einem aus Arzneimittelinformationen, einer Patientenpräferenz und Krankengeschichte des Patienten basiert.

11. Verfahren nach Anspruch 10, ferner umfassend Verwenden von Behandlungsanweisungen und/oder des Behandlungscodes, um eine ePA zu aktualisieren.

12. Verfahren nach Anspruch 10, ferner umfassend Erzeugen von Untersuchungsnotizen zum Befüllen der ePA.

13. Verfahren nach Anspruch 7, ferner umfassend Neuberechnen der Erfolgswahrscheinlichkeit zum Anpassen des Behandlungsplans.

14. Verfahren nach Anspruch 7, wobei die Patientenbehandlungsanweisungen Anforderungen für zusätzliche Untersuchungen umfassen.

15. Verfahren nach Anspruch 14, ferner umfassend Berechnen eines Risikos eines falsch-negativen Ergebnisses für die eine oder mehrere Krankheiten.

## Revendications

1. Système de données médicales pour générer des instructions de traitement de patient, le système comprenant :
une interface de patient (106, 304) avec un équipement médical couplé à une pluralité de capteurs, l'interface étant configurée pour recevoir des données liées au patient en tant qu'entrée directe provenant d'un patient et des mesures d'équipement médical prises par le patient au moyen de l'équipement médical (308), et un signal provenant de la pluralité de capteurs comprenant une position et orientation d'au moins un instrument médical (224) de l'équipement médical lorsque les mesures d'équipement médical sont prises,
dans lequel la pluralité de capteurs comprennent une diode infrarouge (226) et un capteur inertiel de suivi d'équipement de patient (208) fixé à l'instrument médical (224), dans lequel l'interface comprend une caméra à dispositif d'imagerie spectrale (204) couplée à un moniteur (206), dans lequel la caméra à dispositif d'imagerie spectrale (204) est configurée pour afficher par le biais du moniteur (206) la position de l'instrument médical (224) et un signal de référence formé par un signal lumineux émis par la diode infrarouge (226) de manière à permettre au patient de déterminer la position de l'instrument médical (224) et de régler la position par rapport à un emplacement cible affiché par la caméra à dispositif d'imagerie spectrale (204),
dans lequel l'interface est en outre configurée pour générer un score d'exactitude d'usage d'instrument médical représentatif de la fiabilité de la mesure de l'instrument médical sur le patient, le score d'exactitude étant basé sur la position de l'instrument médical (224) et l'emplacement cible, l'interface étant en outre configurée pour déterminer si le score d'exactitude tombe au-dessous d'un seuil et générer une demande de mesure répétée ou une aide à l'usage du dispositif pour positionner l'instrument médical (224) en réponse, l'interface étant en outre configurée pour générer un score de confiance indicatif d'une plage acceptable de l'instrument médical (224) par comparaison des mesures d'équipement médical par rapport à des données de mesure de référence qui seraient attendues de l'instrument médical (224) ; un moteur de diagnostic utilisant un processus d'apprentissage machine pour calculer des données de diagnostic comprenant des probabilités associées à des maladies qui ont été identifiées à partir d'un ensemble de maladies potentielles, dans lequel le score d'exactitude et le score de confiance sont utilisés lors du calcul des données de diagnostic ;
un moteur de traitement couplé au moteur de diagnostic, le moteur de traitement reçoit les données de diagnostic du moteur de diagnostic et réalise les étapes de :
détermination d'une option de traitement pour une ou plusieurs des maladies ;
analyse d'une vraisemblance de succès pour l'option de traitement ;
sur la base de l'analyse, génération d'instructions de traitement de patient pour une ou plusieurs options de traitement ;
réception d'un retour d'informations de patient concernant les instructions de traitement de patient, le retour d'informations de patient comprenant des informations sur un état du patient et une exactitude d'un traitement comme perçue par le patient ; et
fourniture du retour d'informations de patient au moteur de diagnostic pour améliorer les processus d'apprentissage automatique.

2. Système de données médicales selon la revendication 1, dans lequel le moteur de traitement attribue des scores de criticité à une ou plusieurs des maladies.

3. Système de données médicales selon la revendication 1, dans lequel le moteur de diagnostic garnit un dossier de santé électronique (EHR).

4. Système de données médicales selon la revendication 3, comprenant en outre un moteur de génération de notes qui génère des notes d'examen pour garnir l'EHR.

5. Système de données médicales selon la revendication 1, dans lequel le système de données médicales comprend un moteur de codage qui, sur la base d'au moins une d'informations de médicament, d'une préférence de patient, et d'antécédents de patient, génère une sortie qui comprend un code de traitement.

6. Système de données médicales selon la revendication 5, dans lequel le moteur de traitement utilise au moins un du plan de traitement et du code de traitement pour mettre à jour un EHR.

7. Procédé pour générer des instructions de traitement de patient, le procédé comprenant :
- au niveau d'une interface de patient (106, 304) avec un équipement médical couplé à une pluralité de capteurs,
la réception de données liées au patient en tant qu'entrée directe provenant d'un patient et de mesures d'équipement médical prises par le patient au moyen de l'équipement médical, et d'un signal provenant de la pluralité de capteurs comprenant une position et orientation d'un instrument médical de l'équipement médical lorsque les mesures d'équipement médical sont prises, la pluralité de capteurs comprenant une diode infrarouge (226) et un capteur inertiel de suivi d'équipement de patient (208) fixé à l'instrument médical (224) de l'équipement médical,
dans lequel l'interface comprend une caméra à dispositif d'imagerie spectrale (204) couplée à un moniteur (206), dans lequel la caméra à dispositif d'imagerie spectrale (204) affiche par le biais du moniteur (206) la position de l'instrument médical (224) et un signal de référence formé par un signal lumineux émis par la diode infrarouge (226) de manière à permettre au patient de déterminer la position de l'instrument médical (224) et de régler la position par rapport à un emplacement cible affiché par la caméra à dispositif d'imagerie spectrale (204),
dans lequel l'interface génère un score d'exactitude d'usage d'instrument médical représentatif de la fiabilité de la mesure de l'instrument médical sur le patient, le score d'exactitude étant basé sur la position de l'instrument médical (224) et l'emplacement cible, dans lequel l'interface détermine si le score d'exactitude tombe au-dessous d'un seuil et génère une demande de mesure répétée ou une aide à l'usage du dispositif pour positionner l'instrument médical (224) en réponse, l'interface générant en outre un score de confiance indicatif d'une plage acceptable de l'instrument médical (224) par comparaison des mesures d'équipement médical par rapport à des données de mesure de référence qui seraient attendues de l'instrument médical (224) ;
- au niveau d'un moteur de diagnostic, l'utilisation d'un processus d'apprentissage machine pour calculer des données de diagnostic comprenant des probabilités associées à des maladies qui ont été identifiées à partir d'un ensemble de maladies potentielles et l'utilisation du score d'exactitude et du score de confiance lors du calcul des données de diagnostic ;
- au niveau d'un moteur de traitement, la réception des données de diagnostic du moteur de diagnostic ;
sur la base des données de diagnostic, la détermination d'une option de traitement pour une ou plusieurs des maladies ;
l'analyse d'une vraisemblance de succès pour l'option de traitement ;
sur la base de l'analyse, la génération d'instructions de traitement de patient pour une ou plusieurs options de traitement ;
la réception d'un retour d'informations de patient concernant les instructions de traitement de patient, le retour d'informations de patient comprenant des informations sur un état du patient et une exactitude d'un traitement comme perçue par le patient ; et
la fourniture du retour d'informations de patient au moteur de diagnostic pour améliorer les processus d'apprentissage automatique.

8. Procédé selon la revendication 7, dans lequel des scores de criticité peuvent être attribués aux une ou plusieurs des maladies.

9. Procédé selon la revendication 7, comprenant en outre la réception de données liées au patient d'un dossier de santé électronique (EHR).

10. Procédé selon la revendication 7, comprenant en outre, sur la base des données de diagnostic, la génération d'une sortie qui comprend un code de traitement qui est basé sur au moins l'une d'informations de médicament, d'une préférence de patient, et d'antécédents de patient.

11. Procédé selon la revendication 10, comprenant en outre l'utilisation d'au moins l'une des instructions de traitement et du code de traitement pour mettre à jour un EHR.

12. Procédé selon la revendication 10, comprenant en outre la génération de notes d'examen pour charger le EHR.

13. Procédé selon la revendication 7, comprenant en outre le recalcul de la vraisemblance de succès de réglage du plan de traitement.

14. Procédé selon la revendication 7, dans lequel les instructions de traitement du patient comprennent des demandes d'examen additionnel.

15. Procédé selon la revendication 14, comprenant en outre le calcul d'un risque de faux négatif pour les une ou plusieurs maladies.
